Europäisches Patentamt

European Patent Office

Office européen des brevets

(19)

(11) Publication number: **0 288 198**

**A2**

(12) # EUROPEAN PATENT APPLICATION

(21) Application number: 88303297.1

(22) Date of filing: 13.04.88

(51) Int. Cl.⁴: **C12N 15/00 , C07H 21/04 , C12N 5/00 , C12P 21/00 , A61K 37/02 , A61K 39/29 , //(C12N15/00,C12R1:865)**

The applicant has filed a statement in accordance with Rule 28 (4) EPC (issue of a sample only to an expert). Accession numbers of the deposits: IFO 10206, 10337, 10336, 10338, 10426, 10145.

The microorganisms have been deposited with the Institute of Fermentation under numbers IFO 10206, 10337, 10336, 10338, 10426, 10145.

(30) Priority: 20.04.87 JP 98265/87
12.10.87 JP 256885/87

(43) Date of publication of application:
26.10.88 Bulletin 88/43

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Fujisawa, Yukio
31-104,1 Mikagenakamachi 4-chome
Higashinada-ku
Kobe Hyogo 658(JP)
Inventor: Imai, Sachiko
2-8, Kaminishi 2-chome Hirano-ku
Osaka 547(JP)
Inventor: Miyazaki, Takeshi
13-2, Higashitokiwadai 4-chome Toyono-cho
Toyono-gun Osaka 563-01(JP)

(74) Representative: Lewin, John Harvey et al
Elkington and Fife High Holborn House 52/54
High Holborn
London WC1V 6SH(GB)

(54) Production of Peptide.

(57) A peptide having hepatitis B virus surface antigenicity such as S, M or L protein can be excreted outside a eukaryotic cell carrying a recombinant DNA in which a DNA coding for a signal peptide which functions in a eukaryotic cell is bound to the 5'-terminal of a DNA coding for the peptide having hepatitis B virus surface antigenicity by cultivating the eukaryotic cell carrying the recombinant DNA. In particular, the L protein is not only excreted outside the cell but also accumulated within the cell.

The produced peptide having hepatitis B surface antigenicity can be used as vaccine for prevention of hepatitis B virus infection and as an agent for a diagnostic kit.

EP 0 288 198 A2

## PRODUCTION OF PEPTIDE

Industrial Application

This invention relates to production of peptides. In more detail, this invention relates to production of peptides having hepatitis B virus surface antigenicity and to the DNA and eukaryotic cells used in the production.

Brief Description of the Drawings

Fig. 1 shows an DNA sequence coding for the signal peptide of egg white lysozyme.

Fig. 2, Fig. 3 and Fig. 4 illustrate the construction schema of pGLD LP31-RcT, pGLD LP25W and pGLD LP39-RcT, respectively.

Fig. 5 shows an DNA sequence coding for adr type HBsAg L protein (M protein, S protein) and an amino acid sequence thereof, and Fig. 6 shows an DNA sequence coding for adw type HBsAg S protein and an amino acid sequence thereof.

Fig. 7 shows an DNA sequence coding for the signal peptide of egg white lysozyme, and Fig. 8 illustrates the construction scheme of pGLD LIIP39-RcT.

Prior Art and Problems in the Prior Art

Hepatitis B, a viral disease frequently encountered particularly in tropical Africa, Southeast Asia, and Far East, has been suggested by epidemiological studies to cause chronic hepatitis, cirrhosis, and primary hepatocellular carcinoma. The pathogen is hepatitis B virus (HBV), a DNA virus, which is a spherical particle of 42 nm in diameter and called the Dane's particle named after the discoverer. The envelope (env) proteins present in the outer layer include surface antigen (HBsAg) S, M and L proteins, which are classified into subtypes such as adr, adw, ayr and ayw according to the difference in antigenicity. Only adw-and adr-types are found in Japan.

In blood of patients with hepatitis B, small particles and tubular particles are also found in addition to Dane's particles, having the same type HBsAg as that of Dane's particles. It is known also of other viruses that antibodies directed against the viral superficial antigens prevent infection with the virus, and in the case of HBV, hepatitis B vaccine can possibly be produced from HBsAg of HBV. However, HBV infects only human and chimpanzee; attempts to infect the cultured cells have been unsuccessful. Therefore HBsAg is obtainable only from the blood of infected patients, and the available amount of the small particles barely meets the demand for agents used in diagnostic tests, being insufficient for mass production of vaccine.

Recent progress in molecular biology has enabled one to introduce and express DNAs coding for non-bacterial proteins in microorganisms or animal cells. By taking advantage of genetic engineering, HB vaccine has been developed by the expression of genes coding for HBsAg S (P25) protein among the env proteins in yeast [Valenzuela, P. et al., Nature, 298, 347 (1982); Miyanohara, A. et al., Proc. Natl. Acad. Sci. USA, 80, 1 (1983); Hitzeman, R. A. et al., Nucl. Acids Res., 11, 2745 (1983); Harford, N. et al., Developments in Biological Standardization, 54, 125 (1983); Murray, K. et al., EMBO J., 3, 645 (1984); Choo, K. B. et al., Biochem. Biophys. Res. Commun., 131, 160 (1985)]. Recently the gene coding for HBsAg M protein (P31), an env protein, [Itoh, Y, et al., Biochem. Biophys. Res. Commun., 138, 268 (1986); Itoh, Y., & Fujisawa, Y., Biochem. Biophys. Res. Commun., 141, 942 (1986)] and the gene coding for HBsAg L protein (P39), also an env protein, [Deoux, P. et al., Gene, 48, 155 (1986)] have been expressed in yeast. The gene products within the cells are usually isolated only after several steps of purification. If the products are excreted into the media, purification and isolation will be very easy.

It is known that, when S or M gene is expressed in animal cells as the host cells, S protein particles [Liu, C. C. et al., DNA, 1,213 (1982); Moriarty, A. M. et al., Proc. Natl. Acad. Sci. USA, 78, 2606 (1981); Dubois, M. F. et al., Proc. Natl. Acad. Sci. USA, 77, 4549 (1980); Christman, J. K. et al., Proc. Natl. Acad. Sci. USA, 79, 1815 (1982)], and mixed S and M protein particles [Michel, M. L. et al., Proc. Natl. Acad. Sci.

USA, 81, 7708 (1984); Michel., M. L. et al., Bio/Technology, 3, 561 (1985)] are released into the media. However, animal cells are far more disadvantageous becuase of the complicated procedures of cultivation and from the economic view point. If yeast excretes the env proteins into the media, isolation and purification of the immunogens will be expected to be simplified because contamination with materials derived from the cells are greatly reduced only by separation of cells from the culture media. However, there has been no report that yeast transformants release env protein particles into the media.

On the other hand, it is known that in the sera of the patients with manifested acute hepatitis, pre-S antigen-anti-pre-S antibody system is induced prior to induction of HBs antigen-anti-HBs antibody system, HBc antigen-anti-HBc antibody system, and HBe antigen-anti-HBe antibody system [A. Robert Neurath et al., Nature, 315, 154 (1985)]. At present, attention is focused on the role of pre-S antigen and anti-pre-S antibody in establishment of HBV-infection, and onset mechanism and prevention of hepatitis B. Recently it has been established by the studies in chimpanzee that antiserum directed against a pre-S2 peptide in the pre-S region can neutralize HBV [A. Robert Neurath et al., Vaccine, 4, 35 (1986)], and that a pre-S2 peptide vaccine can prevent the onset of acute hepatitis due to HBV [Y. Itoh et al., Proc. Natl. Acad. Sci. USA, 83, 9174 (1986)]. Antibody against a pre-S1 peptide in the pre-S region has been reported to inhibit binding of HBV with cells derived from hepatocellular carcinoma [A. Robert Neurath et al., Cell, 46, 429 (1986)]. As the anti-pre-S antibody has increasingly been regarded as of major importance, development of a new type of hepatitis B vaccine which can induce anti-pre-S antibodies has been expected. Michel et al. [Proc. Natl. Acad. Sci. USA, 81, 7708 (1984)] introduced L protein genes into animal cells (CHO cells) to produce HBsAg particles containing M and S proteins. It is known that yeast into which a modified M protein gene has been introduced can produce HBsAg particles consisting of M proteins (GP37 and GP34) [Y. Itoh & Y. Fujisawa, Biochem. Biophys. Res. Commun., 141, 942 (1986)]. A recent report described that L protein gene was expressed and a protein (P39) of a molecular weight of 39kDa was produced [P. Dehoux et al., Gene, 48, 155 (1986)]. However, the amount of HBsAg produced was as small as about 25 $\mu$g per 1 of culture broth (0.1 -0.2 $\mu$g per 1 mg of total protein) when measured with a commercially available kit Ausria II (manufactured by Abbott Co.). This amount is far smaller than those of HBsAg S protein particles and of HBsAg M protein particles produced by yeast.

## Means to Solve the Problems

The inventors began the studies to develop a method for production of HBV env protein by extracellular excretion by yeast.

Proteins to be excreted are synthesized in membrane-bound polysomes, isolated on the lumenal side of the endoplasmic reticulum, and excreted outside the cell across the Golgi apparatus and secretion granules [Blobel, G. & Dobberstein, B., J. Cell Biol., 67, 835 (1975)]. It has been clarified that genes coding for proteins to be excreted have almost invariably a coding region for the signal peptide bound immediately before the coding region for the said protein [Walter, P. et al., Cell, 38, 5 (1984)]. That is, this signal peptide is supposed to orient the protein to excretion.

However, the env proteins, HBsAg S and M, expressed in animal cells are unique proteins, because they are excreted outside the cells though, unlike other proteins excreted, they exceptionally do not have such a signal peptide. The excretion of the HBsAg proteins has been explained as follows: The proteins are synthesized first as membrane proteins of endoplasmic reticulum, HBsAg monomers assemble on the membrane, followed by budding into the lumen of the membrane and formation of the HBsAg particles. The particles then enter through the Golgi apparatus into the secretion granules and are excreted outside the cells [Eble, B. E. et al., Mol. Cell Biol., 6, 1454 (1986)]. However, extracellular excretion of env protein particles has not been reported in yeast, an eukaryotic cell like animal cells. As the results of investigation with the recombinant DNA technology, it was found that the env protein particles were excreted outside the yeast cells when the inventors used genes including the coding region for respective signal peptide bound immediately before the gene coding for the env protein, i.e. S (P25), M (P31, pre-S2 + S), or L (p39, entire pre-S + S) protein. The inventors also found that when determined the extent of intracellular expression in yeast which has a recombinant DNA in which a coding region for the signal peptide is bound immediately before the L protein gene, surprisingly far higher levels of the antigen were found within the cells as compared with the levels of antigens induced by a recombinant DNA in which no coding region for the signal peptide is bound.

This invention provides a recombinant DNA in which a DNA coding for a signal peptide which functions in a eukaryotic cell is bound to the 5'-terminal of a DNA coding for a peptide having HBsAg antigenicity, a eukaryotic cell carrying the said recombinant DNA, and a method for production of a peptide having HBsAg antigenicity, which comprises cultivating the said eukaryotic cell carrying the said recombinant DNA, accumulating the peptide having HBsAg antigenicity in the culture medium and recovering the said peptide.

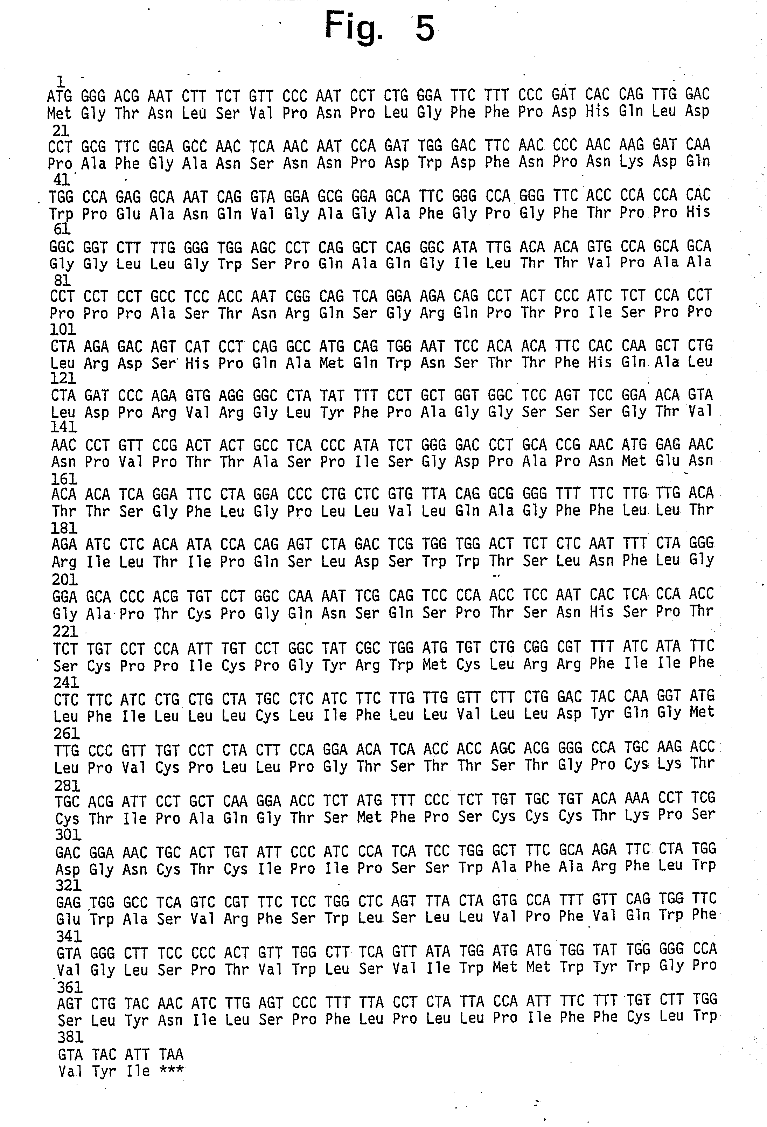

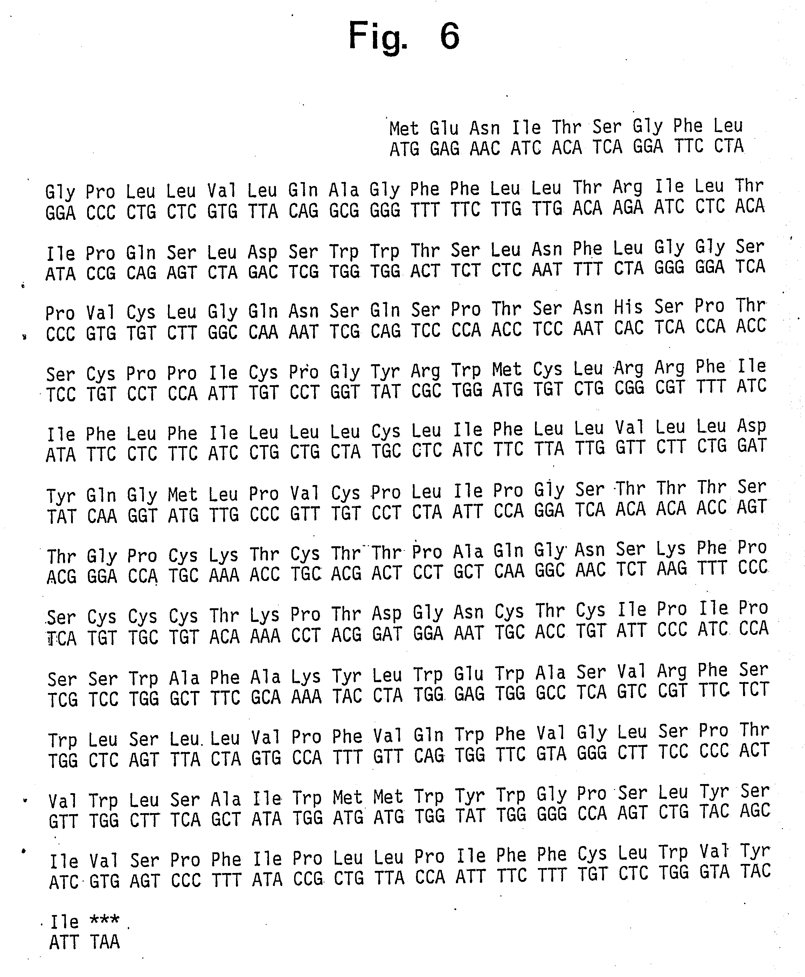

The DNA coding for the peptide having HBsAg antigenicity includes S protein genes, M protein genes and L protein genes of adr, adw, ayw and ayr types. These genes may partly be altered (modifed). When trypsin-like protease-producing yeast is utilized as the host, L protein and M protein may possibly be degraded by the protease; therefore it is desirable that the genes are altered so that the 48th arginine residue from the N-terminal of M protein or a peptide containing the residue (preferably the 44-49 peptide) may be deleted. Preferable genes include L protein gene (altered) coding for the amino acid sequence 1-383 shown in Fig. 5, M protein gene (altered) coding for the amino acid sequence 109-383, and S protein gene coding for the amino acid sequence 158-383 (or the amino acid sequence shown in Fig. 6).

As the DNA coding for the signal peptide, any DNA may be utilized as long as it codes for a signal peptide functioning in a eukaryotic cell, and there are mentioned, for example, signal sequences for mating factor α1 [Brake, A. J. et al., Proc. Natl. Acad. Sci. USA, 81, 4642 (1984); Bitter G. A. et al., Proc. Natl. Acad. Sci. USA, 81, 5330 (1984)], SUC2 and PHO5 [Smith, R. A. et al., Science 229, 1219 (1985)], Killer [Skipper, N. et al., Science, 230, 958 (1985); Baldari, C. et al., EMBO J., 6, 229 (1987)], endoglucanase I [Arsdell, J. N. V. et al., Bio/Technology, 5, 60 (1987)], IFN-α1, IFN-α2 and IFN-γ [Hitzeman, R. A. et al., Science, 219, 620 (1983)], Aspergillus awamori glucoamylase [Innis, M. A. et al., Science, 228, 21 (1985)], mouse immunoglobulin λ and μ [Wood, C. R. et al., Nature, 314, 446 (1985)], chicken lysozyme [Oberto, J. & Davison, J., Gene, 40, 57 (1986)], wheat α-amylase [Rothstein, S. J. et al., Nature, 308, 662 (1984)], and influenza HA [Jabbar, M. A. et al., Proc. Natl. Acad. Sci. USA, 82, 2019 (1985)]. These signal sequences may partly be altered (modified).

A promoter used for expression may be any that can function in a eukaryotic cell, and in yeast preferably are used GLD (GAPH) promoter, PHO5 promoter, PGK promoter, ADH promoter, and PHO81 promoter.

An expression vector may be any that is replicable in a eukaryotic cell and can express the inserted gene. The expression vector in yeast includes pPHO17, pGLD906, and pGLD906-1 [Itoh, Y. et al., Biochem. Biophys. Res. Commum., 138, 268 (1986)].

The eukaryotic cell as the host includes yeast, fungi, and animal cells, among which yeast is preferable. Among species of yeast Saccharomyces cerevisiae is desirable.

The DNA coding for the peptide having HBsAg antigenicity can be prepared from HBV DNA or from the plasmids into which it is inserted, by treatment such as restriction enzyme treatment and ligation with an appropriate adaptor.

The DNA coding for the signal peptide can be prepared enzymatically from a gene containing the said DNA. Because signal peptides are generally composed of about 20 amino acids, the DNA can also easily be synthesized chemically.

The promoter can be prepared enzymatically from the corresponding gene, and can also chemically be synthesized.

The expression vector can be prepared by inserting the promoter into a plasmid replicable in the host. The plasmid replicable in yeast includes pSH19.

The ligation of the DNA coding for the signal peptide to the 5'-terminal of the DNA coding for the peptide having HBsAg antigenicity, and the insertion of the resultant product of the DNA coding for the signal peptide which is bound to the DNA coding for the peptide having HBsAg antigenicity into the 3'-terminal of the promoter in the expression vector can be conducted enzymatically (DNA ligase). The stop codon of the DNA coding for the peptide having HBsAg antigenicity may be followed by a terminator (e.g. PGK terminator) to increase the yield, and a spacer DNA may be inserted between the signal peptide DNA and the HBsAg DNA and between the promoter and the signal peptide DNA. The spacer DNA is preferably composed of less than about 40 bases.

The transformation of a eukaryotic cell with the recombinant DNA (plasmid) can be performed according to the per se known methods.

The resultant transformants are cultivated according to the per se known methods. Culture media for yeast include Burkholder minimum medium [Bostian, K. L. et al., Proc. Natl. Acad. Sci. USA, 77, 4505 (1980)]. Cultivation is usually conducted at 15°C to 40°C, preferably at 24°C to 37°C, for 10 to 96 hours, preferably for 24 to 72 hours, and if necessary under aeration or with shaking.

According to this invention, peptides having HBsAg antigenicity are usually excreted outside the cells are particles containing lipids. Therefore, the supernatant can easily be obtained from the culture broth after cultivation by a per se known method such as centrifugation, and the peptides excreted outside the cell can be purified with an appropriate combination of the procedures usually used for purification of proteins, such as salting out, isoelectric precipitation, gel filtration, ion exchange chromatography, high performance liquid chromatography, affinity chromatography, sucrose gradient ultracentrifugation, and cesium chloride gradient ultracentrifugation.

Particularly L protein is not only excreted outside the cells but also accumulated much within the cells. Therefore the cells are collected with a per se known method after cultivation, and the peptides accumulated within the cells (usually as particles) can be purifed with an appropriate combination of the procedures usually used for purification of proteins, such as cell destruction, salting out, precipitation and fractionation with polyethylene glycol, gel filtration, ion exhcange chromatography, high performance liquid chromatography, affinity chromatography, sucrose gradient ultracentrifugation, and cesium chloride gradient ultracentrifugation.

## Effects

The env proteins obtained in this invention have the same biological activities as those of the known HBsAg particles produced from blood of the patients infected with HBV, and can be utilized as vaccine for prevention of HBV infection similarly as the HBsAg particles and as agents used for diagnostic kits (e.g. an antigen for detection of anti-HBsAg antibody.

The abbreviations of bases and amino acids used in this specification and the drawings are in accordance with the IUPAC-IUB Commision on Biochemical Nomenclature or the conventional abbreviations used in this field. Examples of such abbreviations are listed below. The amino acids showing optical isomerism mean their L-isomers unless otherwise noted.

DNA     deoxyribonucleic acid
RNA     ribonucleic acid
mRNA     messenger RNA
A     Adenine
T     thymine
G     guanine
C     cytosine
dATP     deoxyadenosine triphosphate
dTTP     deoxythymidine triphosphate
dGTP     deoxyguanosine triphosphate
dCTP     deoxycytidine triphosphate
ATP     adenosine triphosphate
EDTA     ethylenediaminetetraacetic acid
SDS     sodium dodecylsulfate
DTT     dithiothreitol
Gly     glycine(G)
Ala     alanine (A)
Val     valine (V)
Leu     leucine (L)
Ile     isoleucine (I)
Ser     serine (S)
Thr     threonine (T)
Cys     cysteine (C)
1/2Cys     half cystine
Met     methionine (M)
Glu     glutamic acid (E)
Asp     aspartic acid (D)
Lys     lysine (K)
Arg     arginine (R)
His     histidine (H)
Phe     phenylalanine (F)
Tyr     tyrosine (Y)
Trp     tryptophan (W)
Pro     proline (P)

Asn    asparagine (N)
Gln    glutamine (Q)
Ap$^r$    ampicillin-resistant gene
Tc$^r$    tetracycline-resistant gene
ars1    autonomous replication sequence 1
IR    inverted repeat

Examples

In the following this invention is illustrated in more detail with examples, but the invention should not be limited only to these.

Deposit of microorganisms carrying the plasmids described in Examples in the depository organizations and the accession numbers are listed in Table 1.

In the table, IFO stands for the Institute of Fermentation, Osaka, and FRI for the Fermentation Research Institute, Agency of Industrial Science and Technology, Ministry of International Trade and Industry, Japan, and S. cerevisiae for Saccharomyces cerevisiae.

## Table 1

| Microorganism | IFO (IFO) | FRI (FERM) |
|---|---|---|
| S. cerevisiae AH22R$^-$/pGLD P31-RcT | 10206 | BP-1059 |
| S. cerevisiae AH22R$^-$/pGLD LP31-RcT | 10337 | BP-1744 (P-9320) |
| S. cerevisiae AH22R$^-$/pGLD LP39-RcT | 10336 | BP-1745 (P-9321) |
| S. cerevisiae AH22R$^-$/pGLD LP25W | 10338 | BP-1746 (P-9323) |
| S. cerevisiae AH22R$^-$/pGLD LIIP39-RcT | 10426 | BP-1747 (P-9648) |

The Sall-digested DNA of PGLD906-1 can also be obtained by Sall digestion of pGLD P31-R. S. cerevisiae AH22R$^-$/pGLD P31-R carrying pGLD P31-R has been deposited with the accession No. IFO 10145 in IFO and No. FERM BP-800 in FRI.

Example 1

Preparation of DNA coding for signal sequence [Xhol-EcoRI]

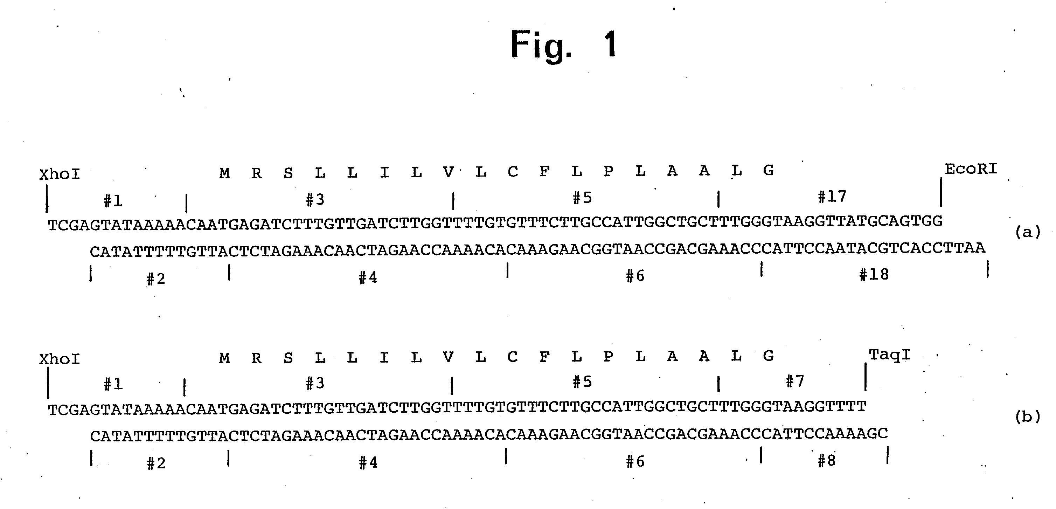

The signal peptide of egg white lysozyme was utilized as the signal sequence for excretion of env protein HBsAg into a medium. A synthetic nucleotide sequence having an Xhol site at the 5′-terminal and an EcoRI site at the 3′-terminal shown in Fig. 1 (a) was used, making reference to the known amino acid sequence [Jung, A. et al., Proc. Natl. Acad. Sci. USA, 77, 5759 (1980)]. The entire sequence comprises 8 oligonucleotide blocks (#1, #2, #3, #4, #5, #6, #17, #18) which were synthesized with the phosphamidide method [Caruthers, M. H. et al., Tetrahedron Letters, 22, 1859 (1981)].

Ten (10) μl (5 μg) each of #2 to #6 and #17 were mixed, to which were added 20 μl of a kinase buffer of a 10-fold concentration [0.5 M Tris-HCl, 0.1 M MgCl$_2$, 0.1 M mercaptoethanol, pH 7.6], 20 μl of 10 mM ATP, 20 μl (50 U) of T4 polynucleotide kinase (manufactured by Takara Shuzo Co. Ltd.), and 80 μl of distilled water. The reaction was conducted at 37°C for 2 hours, and then terminated by treatment at 65°C for 20 minutes. To this reaction mixture, 10 μl (5 μg) each of #1 and #18 were added and then 10 μl of T4 ligase (manufactured by NEB Co.) was further added. The resulting mixture was allowed to react at 14°C overnight. The reaction mixture was subjected to electrophoresis on 6% polyacryl amide gel, and the 80 bp fragment was cut out to extract from the gel by electrophoretic elution, and dissolved in 20 μl of distilled water. One (1) μg of the double-stranded DNA of phage vector M13mp10 [Messing, J., Methods in Enzymol., 101, 20 (1983)] was allowed to react with 10 units of HindIII and 10 units of EcoRI in 20 μl of a reaction medium [50mM Tris-HCl (pH 7.5), 100 mM NaCl, 10 mM MgCl$_2$, 1 mM dithiothreitol] at 37°C for 2 hours, to cleave the said DNA at the HindIII site and the EcoRI site. The said reaction mixture was heat-treated at 65°C for 15 minutes to inactivate the restriction enzymes. To 0.02 μg of the said DNA were added 0.1 μg of the 80 bp DNA fragment described above and 0.5 μg of an synthetic HindIII-XhoI linker consisting of DNA

$$5'\text{-}P\text{-}d \begin{pmatrix} \text{AGCTTGGCC} \\ \\ \text{ACCGGAGCT} \end{pmatrix}$$

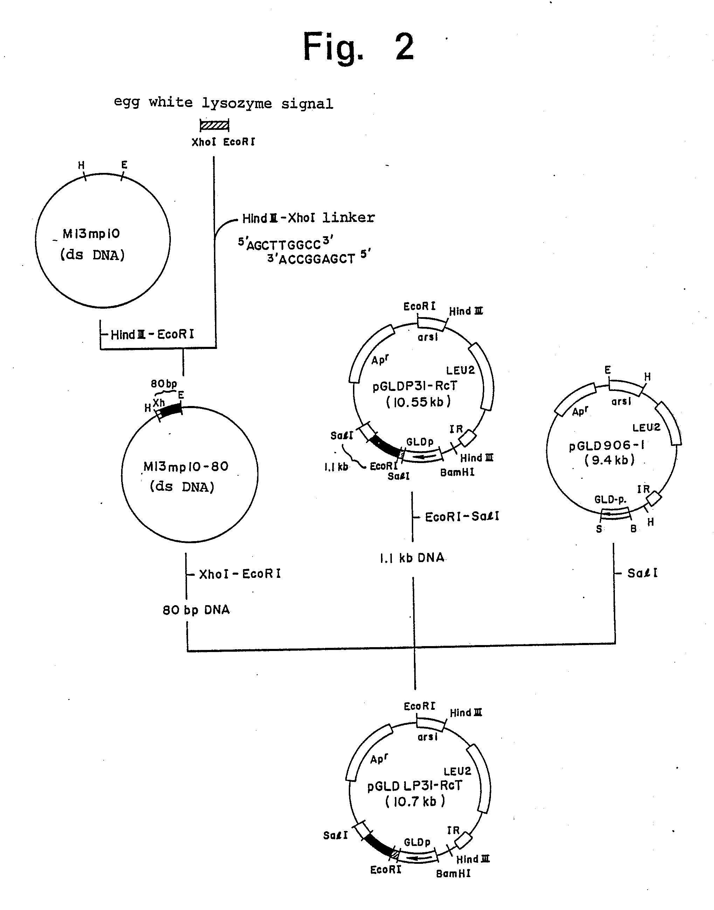

which was synthesized chemically by the phosphamidide method and phosphorylated at the 5'-terminal by T4 polynucleotide kinase. The ligation of the DNAs was performed with T4 DNA ligase in 20 μl of the reaction medium, and then the resultant DNA was introduced into E. coli JM103 by the method of Messing, J. [Methods in Enzymol., 101, 20 (1983)] so that plaques might be formed. From a white plaque, M13mp10-80 in which the 80bp DNA fragment described above was cloned into M13mp10 was obtained. Twenty (20) μg of the double-stranded DNA of M13mp10-80 was allowed to react with 40 units of XhoI and 40 units of EcoRI in 1200 μl of a reaction medium [50 mM Tris-HCl (pH 7.5), 100 mM NaCl, 10 mM MgCl$_2$, 1mM of dithiothreitol] at room temperature overnight, and the reaction mixture was subjected to electrophoresis on 6% polyacrylamide gel. The 80 bp fragment was cut out to extract from the gel by electrophoretic elution, and dissolved in 10 μl of distilled water and kept at -20°C. (Figs. 1 and 2).

Example 2

Construction of expression plasmid for excretion of modified M protein-encoding gene using vector having GLD promoter for expression of foreign genes and transformation of yeast with the said plasmid

Forty (40) μg of the plasmid for expression of the modified M protein (P31), pGLD p31-RcT (described in the Specification of Japanese Patent Application No. 128918/1986), was allowed to react with 100 units of restriction enzymes EcoRI and SalI in 500 μl of a reaction medium [50 mM Tris-HCl (pH 7.5), 100 mM NaCl, 10 mM MgCl$_2$, and 1 mM dithiothreitol] at room temperature overnight, and subjected to electrophoresis on agarose gel, to give a 1.1 kb DNA fragment containing the modified M protein-encoding gene. Zero point five (0.5) μg of the said DNA and 0.5 μg of the DNA coding for the signal sequence obtained in Example 1 were mixed with 0.05 μg of the SalI-digested DNA of the plasmid pGLD 906-1 (the gazette of Japanese Unexamined Patent Publication No. 43991/1986), and the ligation was performed with T4 DNA ligase. E. coli DH1 was transformed with the resulting DNA to give ampicillin-resistant transformants. Among the transformants, the plasmid pGLD LP31-RcT was obtained in which the signal sequence-encoding region and the modified M protein-encoding gene-PGK terminator were inserted down-stream the GLD promoter in the same orientation as the promoter. (Fig. 2).

With pGLD LP31-RcT, the yeast Saccharomyces cerevisiae AH22R⁻ was transformed, to give a transformant (AH22R⁻/pGLD LP31-RcT).

### Example 3

Expression of the modified M protein genes in yeast for excretion

The yeast transformant containing the expression plasmid for excretion of a modified M protein-encoding gene prepared in Example 2 was cultivated with shaking in 5 ml of a culture medium [containing, per 1 $l$, 3 g of $K_2HPO_4$, 50 g of glucose, 4 g of asparagine, 100 mg of L-histidine, 0.1 mg of KI, 500 mg of $MgSO_4$ $7H_2O$, 330 mg of $CaCl_2$ $2H_2O$, 0.4 mg of $CuSO_4$ $5H_2O$, 2.5 mg of $FeSO_4$ $7H_2O$, 0.4 mg of $MnSO_4$ $4H_2O$, 0.2 mg of $(NH_4)_3PO_4$ $12MoO_3$ $3H_2O$, 3.1 mg of $ZnSO_4$ $7H_2O$, 10 mg of inositol, 0.2 mg of thiamine, 0.2 mg of pyridoxine, 0.2 mg of Ca-pantothenate, 0.2 mg of niacin, and 0.002 mg of biotin] at 30°C for 3 days, and 2 ml of the culture broth was transferred into 18 ml of the medium described above, and then the cultivation was conducted at 30°C for 1 day. Then 2 ml of the resultant culture broth was transferred into 18 ml of a fresh culture medium [containing, per 1 $l$, 400 mg of $KH_2PO_4$, 80 g of sucrose, 5 g of asparagine, 300 mg of L-histidine, 2.0 g of KCl, 0.1 mg of KI, 650 mg of $MgSO_4$ $7H_2O$, 429 mg of $CaCl_2$ $2H_2O$, 10 g of glucose, 25 mM of Tris-maleic acid (pH 6.5), 0.4 mg of $CuSO_4$ $5H_2O$, 2.5 mg of $FeSO_4$ $7H_2O$, 0.4 mg of $MnSO_4$ $4H_2O$, 0.2 mg of $(NH_4)_3PO_4$ $12MoO_3$ $3H_2O$, 3.1 mg of $ZnSO_4$ $7H_2O$, 10 mg of inositol, 0.2 mg of thiamine, 0.2 mg of pyridoxine, 4.0 mg of Ca-pantothenate, 4.0 mg of niacin, and 0.040 mg of biotin], and the cultivation was conducted with shaking at 30°C for 3 days. Seventy-two (72) hours later a sample was drawn and centrifuged (10,000 $\times$ g for 10 minutes) to be separated into a supernatant and cells.

The HBsAg antigenicity of the supernatant was determined by using Auszyme II [manufactured by Abbott Co., Ltd.]. The results are shown in Table 2; the amount of HBsAg produced was calculated per 1 $l$ of the broth.

### Example 4

Preparation of DNA coding for the signal sequence [XhoI-TaqI]

The signal peptide of egg white lysozyme was utilized as the signal sequence for excretion of env protein HBsAg into a culture broth. A synthetic nucleotide sequence having an XhoI site at the 5′-terminal and a TaqI site at the 3′-terminal as shown in Fig. 1 (b) was used, making reference to the known amino acid sequence [Jung, A. et al., Proc. Natl. Acad. Sci. USA, 77, 5759 (1980)]. The entire sequence comprises 8 oligonucleotide blocks (#1, #2, #3, #4, #5, #6, #7, #8,) which were synthesized with the phosphamidide method [Caruthers, M. H. et al., Tetrahedron Letters, 22, 1859 (1981)].

Ten (10) $\mu$l (5 $\mu$g) each of #2 to #7 were mixed, to which were added 20 $\mu$l of a kinase buffer of a 10-fold concentration [0.5 M Tris-HCl, 0.1 M $MgCl_2$, 0.1 M mercaptoethanol, pH 7.6], 20 $\mu$l of 10 mM ATP, 20 $\mu$l (50 U) of T4 polynucleotide kinase (manufactured by Takara Shuzo Co., Ltd.), and 80 $\mu$l of distilled water. The reaction was carried out at 37°C for 2 hours, and then terminated by treatment at 65°C for 20 minutes. To this reaction mixture, 10 $\mu$l (5$\mu$g) each of #1 and #8 were added and then 10 $\mu$l of T4 ligase (manufactured by NEB Co.) was further added. The resulting mixture was allowed to react at 14°C overnight. The reaction mixture was subjected to electrophoresis on 6% polyacrylamide gel, and the 76 bp fragment was cut out to extract from the gel by electrophoretic elution, and dissolved in 20 $\mu$l of distilled water.

### Example 5

Construction of expression plasmid for excretion of S protein-encoding gene using vector having GLD promoter for expression of foreign genes and transformation of yeast with the said plasmid

Eighty (80) $\mu$g of the plasmid for expression of the HBsAg S protein (P25), pHBs 51, (gazette of Japanese Unexamined Patent Publication No. 70989/1986), was allowed to react with 200 units of restriction enzyme HpaII in 500 μl of a reaction medium [10 mM Tris-HCl (pH 7.5), 10 mM MgCl$_2$, and 1 mM dithiothreitol] at room temperature overnight, and subjected to electrophoresis on agarose gel, to obtain a 0.815 kb DNA fragment containing the S protein gene. Zero point five (0.5) μg of the said DNA and 5 μg of the DNA coding for the signal sequence obtained in Example 4 were mixed and the ligation was performed with T4 DNA ligase, and the resulting product was allowed to react with 10 units of XhoI and 10 units of HpaII in 40 μl of a reaction medium [50 mM Tris-HCl (pH 7.5), 100 mM NaCl, 10 mM MgCl$_2$, 1 mM dithiothreitol] at 37°C for 2 hours. The reaction mixture was subjected to electrophoresis on agarose gel, to obtain a 0.891 kb S protein-encoding gene fragment containing the signal sequence. One (1) μg of the double-stranded DNA of phage vector M13mp9 [Messing, J., Methods in Enzymol., 101, 20 (1983)] was allowed to react with 10 units of HindIII and 10 units of PstI in 20 μl of a reaction medium [50 mM Tris-HCl (pH 7.5), 100 mM NaCl, 10 mM MgCl$_2$, 1 mM dithiothreitol] at 37°C for 2 hours, to cleave the said DNA at the HindIII site and the PstI site. The said reaction mixture was heat-treated at 65°C for 15 minutes to inactivate the restriction enzymes. To 0.02 μg of the said DNA were added 0.1 μg of the 0.89 kb DNA fragment described above, 0.5 μg of the synthetic HpaII-PstI linker consisting of DNA

$$5'-P-d \begin{pmatrix} CGTGATAAGCTGCA \\ ACTATTCG \end{pmatrix}$$

which was synthesized chemically by the phosphamidide method and phosphorylated at the 5'-terminal by T4 polynucleotide kinase, and 0.5 μg of the synthetic HindIII-XhoI linker used in Example 1 were added, and the ligatation of the DNAs was performed with T4 DNA ligase in 20 μl of the reaction medium, and then the resulting DNA was introduced into E. coli JM103 by the method of Messing, J. [Methods in Enzymol., 101, 20 (1983)] so that plaques might be formed. From a white plaque, M13mp9-LP25 was obtained in which the 0.89 kb DNA fragment was cloned into M13mp9.

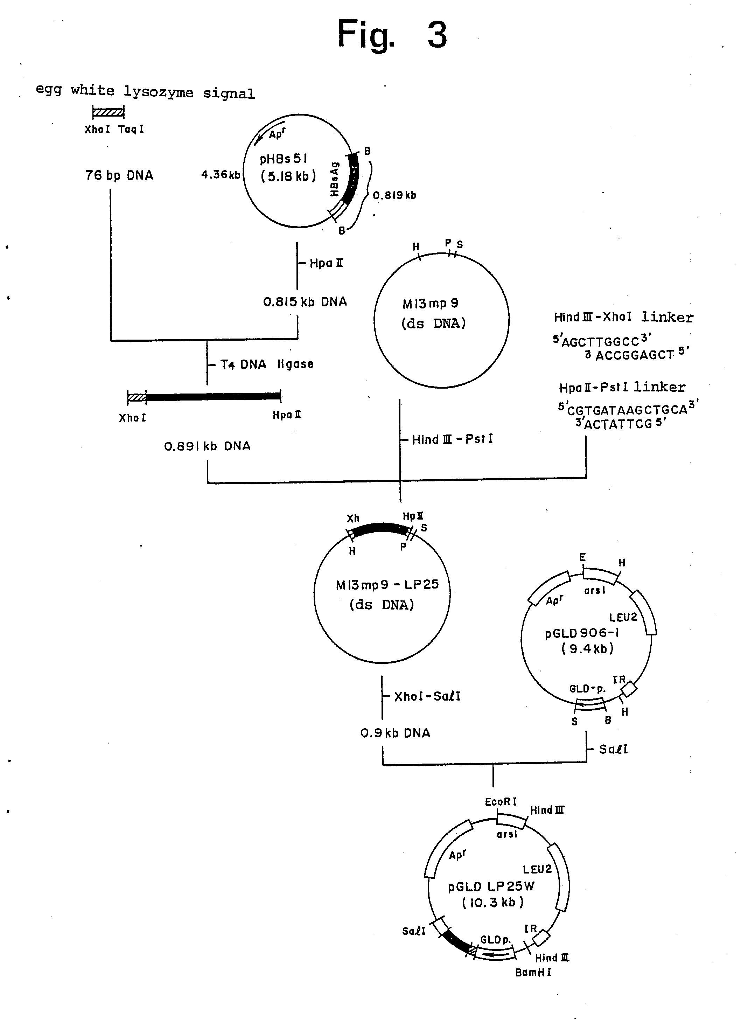

Twenty (20) μg of the double-stranded DNA of M13mp9-LP25 was allowed to react with 40 units of XhoI and 40 units of SalI in 600 μl of a reaction medium [50 mM Tris-HCl (pH 7.5), 100 mM NaCl, 10 mM MgCl$_2$, 1mM dithiothreitol] at 37°C for 4 hours, and the reaction mixture was subjected to electrophoresis on agarose gel, to obtain a 0.9 kb S protein-encoding gene fragment containing the signal sequence. Zero point five (0.5) μg of the said DNA and 0.05 μg of the SalI-digested DNA of the plasmid pGLD 906-1 (the gazette of Japanese Unexamined Patent Publication No. 43991/1986) were mixed, and the ligation was performed with T4 DNA ligase. E. coli DH1 was transformed with the resulting DNA to give ampicillin-resistant transformants. Among the transformant the plasmid pGLD LP25W was obtained in which the signal sequence-encoding region and the S protein-encoding gene-PGK terminator were inserted downstream the GLD promoter in the same orientation as that in the promoter. (Fig. 3).

With pGLD LP25W, the yeast Saccharomyces cerevisiae AH22R⁻ was transformed, to give the transformant (AH22R⁻/pGLD LP25W).

Example 6

Expression of S protein-encoding genes in yeast for excretion

The yeast transformant containing the expression plasmid for excretion of an S protein-encoding gene prepared in Example 5 was cultivated with shaking in 5 ml of a culture medium [containing, per 1ℓ, 3 g of K$_2$HPO$_4$, 50 g of glucose, 4 g of asparagine, 100 mg of L-histidine, 0.1 mg of KI, 500 mg of MgSO$_4$ 7H$_2$O, 330 mg of CaCl$_2$ 2H$_2$O, 0.4 mg of CuSO$_4$ 5H$_2$O, 2.5 mg of FeSO$_4$.7H$_2$O, 0.4 mg of MnSO$_4$ 4H$_2$O, 0.2 mg of (NH$_4$)$_3$PO$_4$ 12MoO$_3$ 3H$_2$O, 3.1 mg of ZnSO$_4$ 7H$_2$O, 10 mg of inositol, 0.2 mg of thiamine, 0.2 mg of pyridoxine, 0.2 mg of Ca-pantothenate, 0.2 mg of niacin, and 0.002 mg of biotin] at 30°C for 3 days, and 0.5 ml of the culture broth was transferred into 4.5 ml of the medium described above, and then the incubation was conducted at 30°C for 1 day. Then 2 ml of the resultant culture broth was transferred into 18 ml of a fresh culture medium [containing, per 1ℓ, 400 mg of KH$_2$PO$_4$, 80 g of sucrose, 5 g of asparagine,

9

300 mg of L-histidine, 2.0 g of KCl, 0.1 mg of KI, 650 mg of $MgSO_4$ $7H_2O$, 429 mg of $CaCl_2$ $2H_2O$, 10 g of glucose, 25 mM of Tris-maleic acid (pH 6.5), 0.4 mg of $CuSO_4$ $5H_2O$, 2.5 mg of $FeSO_4$ $7H_2O$, 0.4 mg of $MnSO_4$ $4H_2O$, 0.2 mg of $(NH_4)_3PO_4$ $12MoO_3$ $3H_2O$, 3.1 mg of $ZnSO_4$ $7H_2O$, 10 mg of inositol, 0.2 mg of thiamine, 0.2 mg of pyridoxine, 4.0 mg of Ca-pantothenate, 4.0 mg of niacin, and 0.040 mg of biotin], and the cultivation was carried out with shaking at 30°C for 3 days. Seventy-two (72) hours later a sample was drawn and centrifuged (10,000 $\times$ g for 10 minutes) to be separated into a supernatant and cells.

The HBsAg antigenicity of the supernatant was determined by using Auszyme II [manufactured by Abbott Co., Ltd.]. The results are shown in Table 2; the amount of HBsAg produced was calculated per 1 $\ell$ of the broth.

Example 7

Construction of expression plasmid for excretion of modified L protein-encoding gene using vector having GLD promoter for expression of foreign genes and transformation of yeast with the said plasmid

Twenty (20) $\mu$g of the plasmid pHBr 330 [Ono, Y. et al., Nucl. Acids Res., 11, 1747 (1983)] was allowed to react with 100 units of restriction enzyme BamHI in 500 $\mu$l of a reaction medium [10 mM Tris-HCl (pH 7.5), 50 mM NaCl, 10 mM $MgCl_2$, and 1 mM dithiothreitol] at 37°C for 2 hours and subjected to electrophoresis on agarose gel, to obtain a 3.2 kb DNA fragment. Ten (10) $\mu$g of the said DNA was allowed to react with 30 units of EcoRI in 20 $\mu$l of a reaction medium [50 mM Tris-HCl (pH 7.5), 100 mM NaCl, 10 mM $MgCl_2$, 1 mM dithiothreitol] at 37°C for 2 hours, and then with 30 units of TaqI at 65°C for 2 hours. The reaction mixture was subjected to electrophoresis on 6% polyacrylamide gel, to give a 0.34 kb DNA fragment. Seven (7) $\mu$g of the said DNA was allowed to ligate with T4 DNA ligase, and allowed to react with 50 units of TaqI in 20 $\mu$l of a reaction medium [50 mM Tris-HCl (pH 7.5), 100 mM NaCl, 10 mM $MgCl_2$, 1 mM dithiothreitol] at 65°C for 3 hours, to give a 0.68 kb DNA fragment. In the presence of 0.5 mM dATP, 0.5 mM dCTP, 0.5 mM dGTP and 0.5 mM dTTP, this fragment was allowed to react with 5 U of Klenow fragment in 100 $\mu$l of a reaction medium [33 mM Tris-acetate (pH 7.9), 66 mM K-acetate, 10 mM Mg-acetate, 100 $\mu$g/ml BSA (bovine serum albumin)] at 37°C for 5 minutes, and then 5 $\mu$l of 0.2 M EDTA was added to stop the reaction, and the deproteinization with phenol-chloroform (1:1) was conducted. There were added 0.5 $\mu$g of an adaptor 5'-P-d(GGAATTCCTCGACC) and 0.5 $\mu$g of 5'-P-d(GGTCGAGGAATTCC) which were obtained by chemical synthesis according to the phosphamidide method and phosphorylated at the 5'-terminal with T4 polynucleotide kinase, and the reaction was conducted in 20 $\mu$l of the reaction medium with T4 DNA ligase to ligate the DNAs, and then the reaction with 30 units of EcoRI was conducted at 37°C for 3 hours (0.36 Kb DNA). One (1) $\mu$g of the double-stranded DNA of phage vector M13mp9 was allowed to react with 10 units of HindIII and 10 units of SalI in 20 $\mu$l of a reaction medium [50 mM Tris-HCl (pH 7.5), 100 mM NaCl, 10 mM $MgCl_2$, 1 mM dithiothreitol] at 37°C for 2 hours, to cleave the said DNA at the HindIII site and the SalI site. The said reaction mixture was heat-treated at 65°C for 15 minutes to inactivate the restriction enzymes. To 0.02 $\mu$g of the said DNA, were added 0.2 $\mu$g of the 0.36 kb DNA fragment described above, 0.5 $\mu$g of the 85 bp fragment obtained by the reaction of M13mp10-80 obtained in Example 1 with restriction enzymes HindIII and EcoRI, and 0.1 $\mu$g of the 1.1 kb DNA fragment obtained by the reaction of the plasmid pGLD P31-RcT (Fig. 2) with restriction enzymes EcoRI and SalI, the ligation of the DNAs was performed with T4 DNA ligase in 20 $\mu$l of the reaction medium, and then the resultant DNA was introduced into E. coli JM103 by the method of Messing, J. [Methods in Enzymol., 101, 20 (1983)] so that plaques might be formed. From a white plaque M13mp9-LP39-RcT was obtained in which the 1.5 kb DNA fragment was cloned into M13mp9.

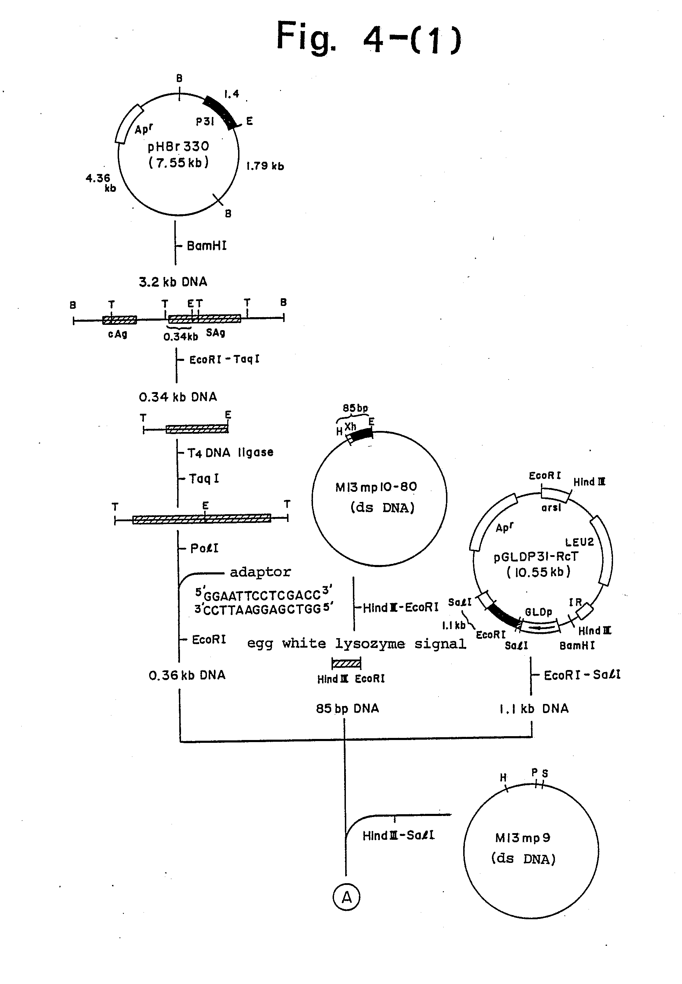

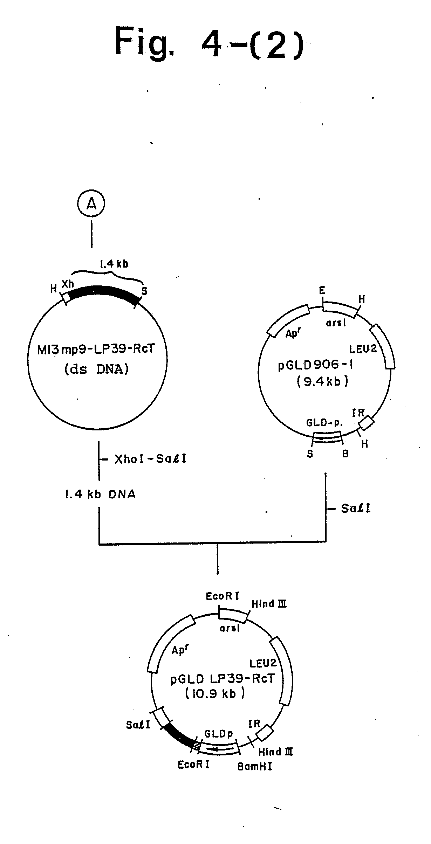

Twenty (20) $\mu$g of the double-stranded DNA of M13mp9-LP39-RcT was allowed to react with 40 units of XhoI and 40 units of SalI in 600 $\mu$l of a reaction medium [50 mM Tris-HCl (pH 7.5), 100 mM NaCl, 10 mM $MgCl_2$, 1 mM dithiothreitol] at 37°C for 4 hours, and the reaction mixture was subjected to electrophoresis on agarose gel, to give the 1.4 kb modified L protein-encoding gene fragment containing the signal sequence. Zero point five (0.5) $\mu$g of the said DNA and 0.05 $\mu$g of the SalI-digested DNA of the plasmid pGLD 906-1 (the gazette of Japanese Unexamined Patent Publication No. 43991/1986) were mixed, and the ligation was performed with T4 DNA ligase. E. coli DH1 was transformed with the resulting DNA to give ampicillin-resistant transformants. Among the transformants the plasmid pGLD LP39-RcT was obtained in which the signal sequence-encoding region and the modified L protein-encoding gene-PGK terminator were inserted downstream the GLD promoter in the same orientation as that in the promoter. (Fig. 4)

With pGLD LP39-RcT, the yeast Saccharomyces cerevisiae AH22R⁻ was transformed, to give the transformant (AH22R⁻/pGLD LP39-RcT).

Example 8

Expression of modified L protein-encoding genes in yeast for excretion

The yeast transformant containing the expression plasmid for excretion of a modified L protein-encoding gene prepared in Example 7 was cultivated with shaking in 5 ml of a culture medium [containing, per 1 $\ell$, 3 g of $K_2HPO_4$, 50 g of glucose, 4 g of asparagine, 100 mg of L-histidine, 0.1 mg of KI, 500 mg of $MgSO_4$ $7H_2O$, 330 mg of $CaCl_2$ $2H_2O$, 0.4 mg of $CuSO_4$ $5H_2O$, 2.5 mg of $FeSO_4$ $7H_2O$, 0.4 mg of $MnSO_4$ $4H_2O$, 0.2 mg of $(NH_4)_3PO_4$ $12MoO_3$ $3H_2O$, 3.1 mg of $ZnSO_4$ $7H_2O$, 10 mg of inositol, 0.2 mg of thiamine, 0.2 mg of pyridoxine, 0.2 mg of Ca-pantothenate, 0.2 mg of niacin, and 0.002 mg of biotin] at 30°C for 3 days, and 0.5 ml of the culture broth was transferred into 4.5 ml of the medium described above, and the cultivation was conducted at 30°C for 1 day. Then 2 ml of the resultant culture broth was transferred into 18 ml of a fresh culture medium [containing, per 1 $\ell$, 400 mg of $KH_2PO_4$, 80 g of sucrose, 5 g of asparagine, 300 mg of L-histidine, 2.0 g of KCl, 0.1 mg of KI, 650 mg of $MgSO_4$ $7H_2O$, 429 mg of $CaCl_2$ $2H_2O$, 10 g of glucose, 25 mM of Tris-maleic acid (pH 6.5), 0.4 mg of $CuSO_4$ $5H_2O$, 2.5 mg of $FeSO_4$ $7H_2O$, 0.4 mg of $MnSO_4$ $4H_2O$, 0.2 mg of $(NH_4)_3PO_4$ $12MoO_3$ $3H_2O$, 3.1 mg of $ZnSO_4$ $7H_2O$, 10 mg of inositol, 0.2 mg of thiamine, 0.2 mg of pyridoxine, 4.0 mg of Ca-pantothenate, 4.0 mg of niacin, and 0.040 mg of biotin], and the cultivation was conducted with shaking at 30°C for 3 days. Seventy-two (72) hours later a sample was drawn and centrifuged (10,000 $^\times$ g for 10 minutes) to be separated into a supernatant and cells.

The HBsAg antigenicity of the supernatant was determined by using Auszyme II [manufactured by Abbott Co., Ltd.]. The results are shown in Table 2; the amount of HBsAg produced was calculated per 1 $\ell$ of the broth.

The HBsAg antigenicity of the cells obtained was determined by using Auszyme II; the amount of HBsAg produced was about 50 mg per 1 $\ell$ of the broth.

Table 2

| Yeast transformant | Excreted HBsAg (μg/1$\ell$ broth) |
|---|---|
| Saccharomyces cerevisiae AH22R⁻/pGLD LP31-RcT | 350 |
| Saccharomyces cerevisiae AH22R⁻/pGLD LP25W | 70 |
| Saccharomyces cerevisiae AH22R⁻/pGLD LP39-RcT | 310 |

Example 9

Preparation of DNA coding for the signal sequence [XhoI-TaqI type II]

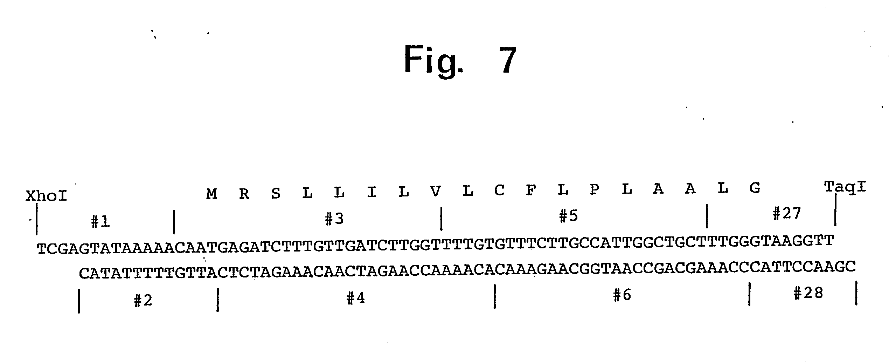

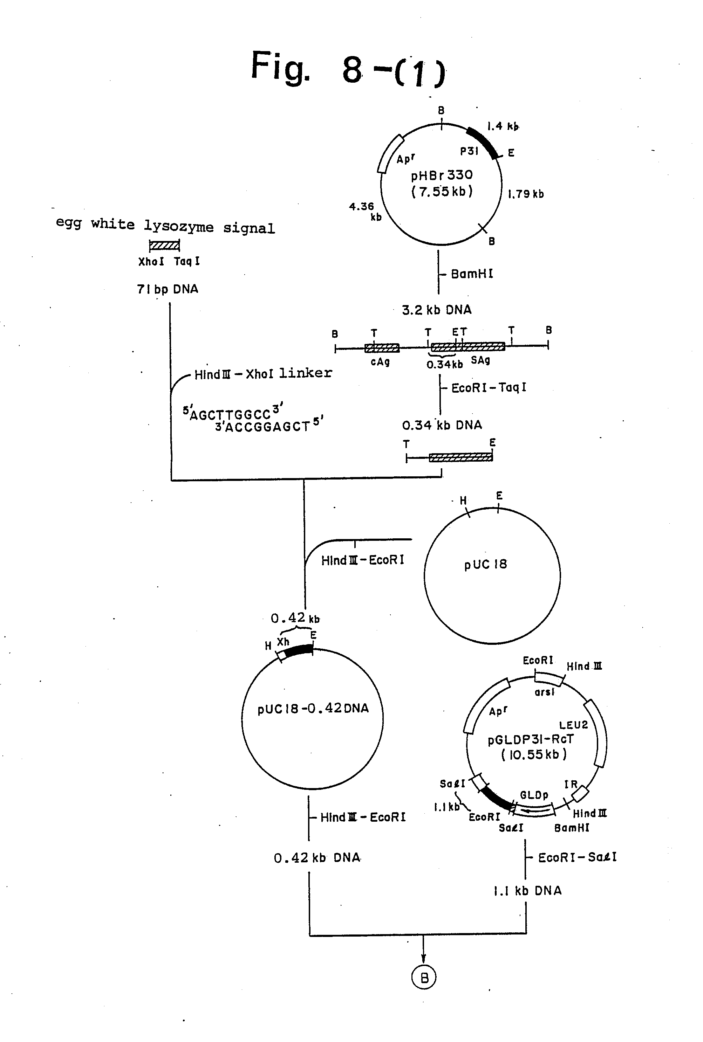

The signal peptide of egg white lysozyme was utilized as the signal sequence for excretion of env protein HBsAg into culture media. A synthetic nucleotide sequence having an XhoI site at the 5′-terminal and a TaqI site at the 3′-terminal as shown in Fig. 7 was used, making reference to the known amino acid sequence [Jung, A. et al., Proc. Natl. Acad. Sci. USA, 77, 5759 (1980)]. The entire sequence comprises 8 oligonucleotide blocks (#1, #2, #3, #4, #5, #6, #27, #28) which were synthesized with the phosphamidide method [Caruthers, M. H. et al., Tetrahedron Letters, 22, 1859 (1981)].

Ten (10) μl (5 μg) each of #2 to #6 and #27 were mixed, to which were added 20 μl of a kinase buffer of a 10-fold concentration [0.5 M Tris-HCl, 0.1 M MgCl$_2$, 0.1 M mercaptoethanol, pH 7.6], 20 μl of 10 mM ATP, 20 μl (50 U) of T4 polynucleotide kinase (manufactured by Takara Shuzo Co., Ltd.), and 80 μl of distilled water. The reaction was conducted at 37°C for 2 hours, and then terminated by treatment at 65°C for 20 minutes. To this reaction mixture 10 μl (5 μg) each of #1 and #28 were added and then 10 μl of T4 ligase (manufactured by NEB Co.) was further added. The resulting mixture was allowed to react at 14°C overnight. The reaction mixture was subjected to electrophoresis on 6% polyacrylamide gel, and the 71 bp fragment was cut out to extract from the gel by electrophoretic elution, and dissolved in 20 μl of distilled water.

Example 10

Construction of expression plasmid for excretion of modified L protein-encoding gene using vector having GLD promoter for expression of foreign genes and transformation of yeast by the said plasmid

Twenty μg (20) of the plasmid pHBr330 [Ono, Y. et al., Nucl. Acids Res., 11, 1747 (1983)] was allowed to react with 100 units of restriction enzyme BamHI in 600 μl of a reaction medium [10 mM Tris-HCl (pH 7.5), 50 mM NaCl, 10 mM MgCl$_2$, and 1 mM dithiothreitol] at 37°C for 2 hours and then subjected to electrophoresis on agarose gel, to obtain a 3.2 kb DNA fragment. Ten (10) μg of the said DNA was allowed to react with 30 units of EcoRI in 20 μl of a reaction medium [50 mM Tris-HCl (pH 7.5), 100 mM NaCl, 10 mM MgCl$_2$, 1 mM dithiothreitol] at 37°C for 2 hours, and then with 30 units of TaqI at 65°C for 2 hours. The reaction mixture was subjected to electrophoresis on 6% polyacrylamide gel, to give a 0.34 kb DNA fragment. Two (2) μg of the DNA of plasmid vector pUC18 was allowed to react with 10 units of HindIII and 10 units of EcoRI in 20 μl of a reaction medium [50 mM Tris-HCl (pH 7.5), 10 mM NaCl, 10 mM MgCl$_2$, 1 mM dithiothreitol] at 37°C overnight, to cleave the said DNA at the HindIII site and the EcoRI site, and kept at -20°C. To 0.04 μg of the said DNA, were added 0.2 μg of the 0.34 kb DNA fragment described above, 0.5 μg of the 71 bp fragment obtained in Example 9, and 0.5 μg of the synthetic HindIII-XhoI linker used in Example 1, and the ligation of the DNAs was performed with T4 DNA ligase in 20 μl of the reaction medium, and then the resultant DNA was introduced into E. coli JM103 by the method of Messing, J. [Methods in Enzymol., 101, 20 (1983)] so that colonies might be formed. From a white colony pUC18-0.42 DNA was obtained in which the 0.42 kb DNA fragment was cloned into pUC18.

Twenty (20) μg of the DNA of pUC18-0.42 DNA was allowed to react with 40 units of HindIII and 40 units of EcoRI in 800 μl of a reaction medium [50 mM Tris-HCl (pH 7.5), 10 mM NaCl, 10 mM MgCl$_2$, 1mM dithiothreitol] at 37°C overnight, and the reaction mixture was subjected to electrophoresis on agarose gel, to give the 0.42 kb DNA fragment. Two (2) μg of the DNA of the plasmid vector pUC18 was allowed to react with 10 units of HindIII in 20 μl of a reaction medium [50 mM Tris-HCl (pH 7.5), 10 mM NaCl, 10 mM MgCl$_2$, 1 mM dithiothreitol] at 37°C overnight. The concentration of NaCl in the reaction mixture was made to 150 mM, and the reaction with 10 units of SalI was carried out at 37°C overnight to cleave the said DNA at the Hind III site and SalI site, and the resulting DNA was kept at -20°C. To 0.04 μg of the said DNA, were added 0.5 μg of the 0.42 kb DNA fragment described above, and 0.1 μg of the 1.1 kb DNA fragment obtained by the reaction of plasmid pGLD p31-RcT with restriction enzymes EcoRI and SalI, and the ligation of the DNAs was performed with T4 DNA ligase in 20 μl of the reaction medium, and then the resultant DNA introduced into E. coli JM103 by the method of Messing, J. [Methods in Enzymol., 101, 20 (1983)] so that colonies might be formed. From a white colony pUC18-LII39-RcT was obtained in which the 1.5 kb DNA fragment was cloned into pUC18.

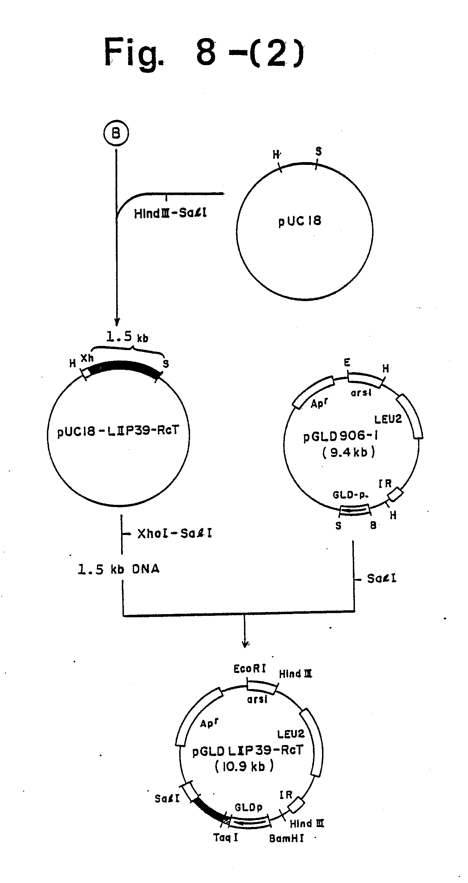

Twenty (20) μg of the DNA of pUC18-LIIP39-RcT was allowed to react with 40 units of XhoI and 40 units of SalI in 600 μl of a reaction medium [50 mM Tris-HCl (pH 7.5), 100 mM NaCl, 10 mM MgCl$_2$, 1mM dithiothreitol] at 37°C for 5 hours, and the reaction mixture was subjected to electrophoresis on agarose gel, to give the 1.5 kb modified L protein-encoding gene fragment containing the signal sequence. Zero point five (0.5) μg of the said DNA and 0.05 μg of SalI-digested DNA of the plasmid pGLD 906-1 (the gazette of Japanese Unexamined Patent Publication No. 43991/1986) were mixed, and the ligation of the DNAs was performed with T4 DNA ligase. E. coli DH1 was transformed with the resulting DNA to give ampicillin-resistant transformants. Among the transformants the plasmid pGLD LIIP39-RcT in which the signal sequence-encoding region and the modified L protein-encoding gene-PGK terminator were inserted downstream the GLD promoter in the same orientation as that in the promoter was obtained. (Fig. 8).

With pGLD LIIP39-RcT, the yeast Saccaromyces cerevisiae AH22R⁻ was transformed, to give the transformant (AH22R⁻/pGLD LIIP39-RcT).

Example 11

Expression of modified L protein-encoding genes in yeast

The yeast transformant containing the plasmid for expression of a modifed L protein-encoding gene prepared in Example 10 was cultivated with shaking in 5 ml of a culture medium [containing, per 1 $\ell$, 3 g of $K_2HPO_4$, 50 g of glucose, 4 g of asparagine, 100 mg of L-histidine, 0.1 mg of KI, 500 mg of $MgSO_4$ $7H_2O$, 330 mg of $CaCl_2$ $2H_2O$, 0.4 mg of $CuSO_4$ $5H_2O$, 2.5 mg of $FeSO_4$ $7H_2O$, 0.4 mg of $MnSO_4$ $4H_2O$, 0.2 mg of $(NH_4)_3PO_4$ $12MoO_3$ $3H_2O$, 3.1 mg of $ZnSO_4$ $7H_2O$, 10 mg of inositol, 0.2 mg of thiamine, 0.2 mg of pyridoxine, 0.2 mg of Ca-pantothenate, 0.2 mg of niacin, and 0.002 mg of biothin] at 30°C for 3 days, and 0.5 ml of the culture broth was transferred into 4.5 ml of the medium described above, and then the cultivation was conducted at 30°C for 1 day. Then 2 ml of the resultant culture broth was transferred into 18 ml of a fresh culture medium [containing, per 1 $\ell$, 400 mg of $KH_2PO_4$, 80 g of sucrose, 5 g of asparagine, 300 mg of L-histidine, 2.0 g of KCl, 0.1 mg of KI, 650 mg of $MgSO_4$ $7H_2O$, 429 mg of $CaCl_2$ $2H_2O$, 10 g of glucose, 25 mM of Tris-maleic acid (pH 6.5), 0.4 mg of $CuSO_4$ $5H_2O$, 2.5 mg of $FeSO_4$ $7H_2O$, 0.4 mg of $MnSO_4$ $4H_2O$, 0.2 mg of $(N_4)_3PO_4$ $12MoO_3$ $3H_2O$, 3.1 mg of $ZnSO_4$ $7H_2O$, 10 mg of inositol, 0.2 mg of thiamine, 0.2 mg of pyridoxine, 4.0 mg of Ca-pantothenate, 4.0 mg of niacin, and 0.040 mg of biotin], and the cultivation was conducted with shaking at 30°C. Forty-eight (48) hours later a sample was drawn and centrifuged (10,000 × g for 10 minutes) to be separated into a supernatant and cells.

The HBsAg antigenicity of the cells obtained was determined by using Auszyme II [manufactured by Abbott Co., Ltd.]; the amount of HBsAg produced was about 50 mg per 1 $\ell$ of the broth.

Example 12

Purification of modified L protein

(1) Extraction from Cells

A hundred and fifty (150) g of the preserved cells under freezing of yeast S. cerevisiae AH22R⁻/pGLD LIIP39-RcT obtained by cultivating the cells according to the method described in Example 11 and freezing them at -20°C were uniformly suspended in 600 ml of a buffer (pH 7.2) containing 0.1% polyoxyethylene (20) sorbitan monooleate (Tween 80)-7.5 M urea-15 mM tetrasodium ethylenediaminetetraacetate (EDTA)-2 mM phenylmethylsulfonyl fluoride (PMSF)-0.1 mM (p-amidinophenyl)-methanesulfonyl fluoride hydrochloride (P-APMSF)-100 mM sodium phosphate, and the suspension was treated in ice by Bead-Beater (Biospec Products, USA) with glass beads (0.50-0.75 mm in diameter) for 6 minutes to disrupt the cells. This extract was centrifuged at 12,000 × g for 30 minutes to obtain 540 ml of a supernatant.

(2) Fractionation by polyethyleneglycol

To the supernatant obtained above, there was gradually added a 0.75 volume of 33% (w/w) polyethylene-glycol 6000 (PEG-6000), and the pH value was adjusted to 6.0. The mixture was stirred for 30 minutes, and then centrifuged at 13,900 × g for 30 minutes to recover the fraction of HBsAg as a precipitate. The precipitate was dissolved in 200 ml of a buffer (pH 7.2) containing 7.5 M urea-15 mM EDTA-2 mM PMSF-0.1 mM P-APMSF-100 mM sodium phosphate with stirring at 4°C overnight.

## (3) Cesium chloride (CsCl) gradient ultracentrifugation

In a ultracentrifuge tube for Beckman SW-28, there were layered 4 ml of 40% cesium chloride (CsCl)-5 M urea-2 mM EDTA-1 mM PMSF-0.05 mM P-APMSF-10 mM potassium phosphate buffer (pH 7.4), 6 ml of 30% CsCl-5 M urea-2 mM EDTA-1 mM PMSF-0.05 mM P-APMSF-10 mM potassium phosphate buffer (pH 7.4), 7 ml of 20% CsCl-5 M urea-2 mM EDTA-1 mM PMSF-0.05 mM P-APMSF-10 mM potassium phosphate buffer (pH 7.4), 8 ml of 10% CsCl-5 M urea-2 mM EDTA-1 mM PMSF-0.05 mM P-APMSF-10 mM potassium phosphate buffer (pH 7.4) and 13 ml of the solution described above, and the ultracentrifugation was carried out at 28,000 rpm at 4°C for 16 hours to concentrate and purify the HBsAg modified L protein in vicinity of a density of 1.2.

The concentrated and purified HBsAg fraction obtained by the above ultracentrifugation was dialysed against a buffer (pH 7.2) containing 7.5 M urea-15 mM EDTA-2 mM PMSF-0.1 mM P-APMSF-100 mM sodium phosphate, and the thus obtained solution was again fractionated by the CsCl gradient ultracentrifugation described above. The concentrated and purifed HBsAg fraction was dialysed against PBS (8 g/l of NaCl, 2.9g/$l$ of Na$_2$HPO$_4$ 12H$_2$O, 0.2 g/l of KH$_2$PO$_4$, 0.2 g /l of KCl) -0.1 mM P-APMSF to give 31 ml of an HBsAg modifed L protein solution.

## (4) Sucrose gradient ultracentrifugation

In a ultracentrifuge tube for Beckman SW-28, there were layered 6 ml of 50% sucrose-2 mM EDTA-0.05 mM P-APMSF-10 mM potassium phosphate buffer (pH 7.4), 6 ml of 40% sucrose-2 mM EDTA-0.05 mM P-APMSF-10 mM potassium phosphate buffer (pH 7.4), 6 ml of 30% sucrose-2 mM EDTA-0.05 mM P-APMSF-10 mM potassium phsphate buffer (pH 7.4), 6 ml of 20% sucrose-2 mM EDTA-0.05 mM P-APMSF10 mM potassium phosphate buffer (pH 7.4), 10% sucrose-2 mM EDTA-0.05 mM P-APMSF-10 mM potassium phosphate buffer (pH 7.4) and 7 ml of the HBsAg modified L protein solution described in above (3), and the ultracentrifugation was carried out at 28,000 rpm at 4°C for 16 hours to concentrate and purify the HBsAg modified L protein in vicinity of a sucrose concentration of 35%.

The concentrated and purifed HBsAg modified L protein fraction obtained by the above ultracentrifugation was dialysed against PBS-0.1 mM P-APMSF, and the obtained solution was again fractionated by the sucrose gradient ultracentrifugation described above and dialysed to give 26 ml of an HBsAg modified L protein solution.

## (5) Gel filtration by Sephacyl S-400

A Sephacryl S-400 (Pharmacia, Sweden) column (1.8 $\times$ 85 cm, 210 ml) equilibrated with PBS was charged with the HBsAg modified L protein solution obtained in above (4), and the elution was conducted with the same buffer to collect 80 ml of the HBsAg modified L protein fraction. The eluate was concentrated to give 5.0 ml of the purified sample of HBsAg modified L protein having a protein concentration of 335 $\mu$g/ml.

Example 13

Properties of modified L protein

The HBsAg modified L protein obtained in Example 12 was investigated on the following properties.

14

(1) Molecular weight

As a result of SDS-polyacrylamide slab gel electrophoresis according to the method of Laemmli [Nature, 227, 680 (1970)] and silver-staining, the HBsAg modified L protein was composed of glycoproteins having a molecular weight of about 49,000 and 52,000.

(2) N-Terminal amino acid sequence

The N-terminal amino acid sequence was analysed by appling the automatic Edman degradation method using a Gas-phase Protein Sequenator (Applied Biosystems, Model 470A, USA) to 2.2 mmol of the HBsAg modified L protein. Phenylthiohydantoin-amino acids (PTH-amino acids) were identified by high-performance liquid chromatography using a Micro Pak SP-ODS (Varian, USA) column. The PTH-amino acids detected in the respective steps are shown in Table 3.

Table 3

| Step | Amino acid mainly detected |
|------|----------------------------|
| 1    | Lys                        |
| 2    | Val                        |
| 3    | X                          |
| 4    | Gln                        |
| 5    | Gly                        |
| 6    | Met                        |
| 7    | Gly                        |
| 8    | Thr                        |
| 9    | X                          |
| 10   | Leu                        |
| 11   | X                          |
| 12   | Val                        |
| 13   | Pro                        |
| 14   | Asn                        |
| 15   | Pro                        |
| 16   | Leu                        |
| 17   | Gly                        |
| 18   | Phe                        |
| 19   | Phe                        |
| 20   | Pro                        |

X: unidentified amino acid

(3) Observation by electron microscope

As a result of observation on the HBsAg modified L protein by an electron microscope (Nippon Denki, Model 1200E), there were observed spherical particles having a diameter of 23.3±3.3 nm. In addition to the particles, there were also observed tubular particles mainly having a minor axis of 12.7±1.1 nm and a major axis of about 40 to 120 nm.

(4) C-Terminal amino acid sequence

As a result of the analysis for the C-terminal amino acid using 2.04 nmol of the HBsAg modified L protein, it had isoleucine as the C-terminal amino acid.

Example 14

Purification of modified L protein

The extraction and purification of the HBsAg modifed L protein were conducted according to the method described in Example 12 using 210 g of the preserved cells under freezing of yeast S. cerevisiae AH22R⁻/pGLD LP39-RcT obtained by cultivating the cells according to the method described in Example 8 and freezing them at -20°C, so that 3.5 ml of the purified sample of the HBsAg modified L protein having a protein concentration of 455 μg/ml.

Example 15

Properties of modified L protein

The HBsAg modified L protein obtained in Example 14 was investigated on the following properties.

(1) Molecular weight

As a result of the analysis for molecular weight according to the method described in Example 13 (1), the HBsAg modified L protein was composed of proteins having a molecular weight of 50,000 and 52,000.

(2) N-Terminal amino acid sequence

The results of analysis of 2.7 nmol of the HBsAg modified L protein according to the method described in Example 13 (2) are shown in Table 4.

Table 4

| Step | Amino acid mainly detected |
|------|---------------------------|
| 1 | Lys |
| 2 | Val |
| 3 | Met |
| 4 | Glu |
| 5 | Trp |
| 6 | Asn |
| 7 | X |
| 8 | X |
| 9 | X |
| 10 | X |
| 11 | Gln |
| 12 | Gly |
| 13 | Met |
| 14 | Gly |
| 15 | Thr |
| 16 | X |
| 17 | Leu |
| 18 | X |
| 19 | Val |
| 20 | Pro |

X: unidentified amino acid

(3) Observation by electron microscope

As a result of observation on the HBsAg modified L protein by an electron microscope (Nippon Denki, Model 1200E), there were observed spherical particles having a diameter of 24.6±3.9 nm. In addition to the particles, there were also observed tubular particles mainly having a minor axis of 13.2±1.7 nm and a major axis of about 40 to 130 nm.

(4) C-Terminal amino acid

As a result of the analysis for the C-terminal amino acid using 2.15 nmol of the HBsAg modified L protein, it had isoleucine as the C-terminal amino acid.

Example 16

Purification of modified M protein

The culture broth described in Example 3 was centrifuged at 13,900 × g at 4°C for 10 minutes to obtain 10 ℓ of a supernatant. After the supernatant was saturated with ammonium sulfate to a concentration of 60%, the mixture was centrifuged at 12,000 × g for 20 minutes to recover the HBsAg modified M protein fraction as a precipitate. The obtained precipitate was suspended in 70 ml of 25 mM sodium phosphate buffer (pH 6.0), and the suspension was dialysed against the same buffer. The obtained solution was centrifuged at 27,000 × g for 10 minutes to obtain 70 ml of a supernatant.

(1) Antibody column

Seventy (70) ml of the supernatant was passed through a Formyl-Cellulofine column (10 ml) equilibrated with 25 mM sodium phosphate buffer (pH 6.0) to which mouse-derived anti-HBsAg antibody described in Reference Examples 1 to 3 of International Application No. PCT/JP85/00161 [Japanese Patent Application No. 4092/1986, filed January 10, 1986] was bound. The HBsAg-adsorbed column was washed with 1 M ammonium thiocyanate-10 mM sodium phosphate buffer (pH 6.0) and the elution was performed with 4 M ammonium thiocyanate-10 mM sodium phosphate buffer (pH 6.0). The eluate was concentrated to a volume of 2.2 ml.

(2) Gel filtration by Sephacyl S-300

A Sephacyl S-300 (Pharmacia, Sweden) column (1.8 × 74 cm, 3 ml) equilibrated with 0.85% sodium chloride-20 mM potassium phosphate buffer (pH 7.5) was charged with the concentrate obtained above, and the elution was performed with the same buffer. The eluate was concentrated to obtain 1.5 ml of the purified HBsAg modified M protein sample having a protein concentration of 15 μg/ml.

Example 17

Properties of modified M protein

The HBsAg modified M protein obtained in Example 16 was investigated on the following properties.

(1) Molecular weight

As a result of SDS-polyacrylamide slab gel electrophoresis according to the method of Laemmli [Nature, 227, 680 (1970)] and silver-staining, the HBsAg modified M protein was composed of proteins having a molecular weight of about 34,000, 31,000, 28,000, 24,000, 17,000 and 14,000.

(2) Observation by electron microscope

As a result of observation on the HBsAg modified M protein by an electron microscope [Nippon Denki, Model 1200E], there were observed spherical particles having a diameter of 19.4±2.3 nm.

The spacer-DNA sequences between the signal peptide DNA and the HBsAg DNA in the plasmids are shown in the following.

(1) pGLD LP39-RcT spacer sequence
AAG GTT ATG CAG TGG AAT TCC TCG ACC CGA
Lys Val Met Gln Trp Asn Ser Ser Thr Arg

CAA GGC
Gln Gly

(2) pGLD LP31-RcT spacer sequence
AAG GTT
Lys Val

(3) pGLD LP25W spacer sequence
AAG GTT TTC GGG ATC
Lys Val Phe Gly Ile

(4) pGLD LIIP39-RcT spacer sequence
AAG GTT CGA CAA GGC
Lys Val Arg Gln Gly

(Effect of the invention)

According to this invention, the peptides having HBsAg antigenicity are excreted outside the cells and easily purified. Particularly the L protein is excreted outside the cells and also accumulated abundantly within the cells. Thus mass production of the L protein has become possible.

**Claims**

1. A recombinant DNA in which a DNA coding for a signal peptide which functions in a eukaryotic cell is bound to the 5′-terminal of a DNA coding for a peptide having hepatitis B virus surface antigenicity.

2. A recombinant DNA according to claim 1, wherein the signal peptide is a signal peptide of egg white lysozyme.

3. A recombinant DNA according to claim 1, wherein the peptide having hepatitis B virus surface antigenicity is a peptide having hepatitis B virus S antigenicity, a peptide having hepatitis B virus pre-S2 and S antigenicity, or a peptide having hepatitis B virus pre-S1, pre-S2 and S antigenicity.

4. A recombinant DNA according to claim 1, wherein the peptide having hepatitis B virus surface antigenicity is a peptide having hepatitis B virus pre-S1, pre-S2 and S antigenicity.

5. A recombinant DNA according to claim 1, which is a plasmid replicable in a yeast cell.

6. A recombinant DNA according to claim 1, which is pGLD LP31-RcT, pGLD LP39-RcT, pGLD LP25W or pGLD LIIP39-RcT.

7. A recombinant DNA according to claim 1, which is pGLD LP39-RcT or pGLD LIIP39-RcT.

8. A eukaryotic cell which is transformed with a recombinant DNA in which a DNA coding for a signal peptide which functions in a eukaryotic cell is bound to the 5′-terminal of a DNA coding for a peptide having hepatitis B virus surface antigenicity.

9. A eukaryotic cell according to claim 8, which is a yeast cell.

10. A eukaryotic cell according to claim 8, wherein the signal peptide is a signal peptide of egg white lysozyme.

11. A eukaryotic cell according to claim 8, wherein the peptide having hepatitis B virus surface antigenicity is a peptide having hepatitis B virus S antigenicity, a peptide having hepatitis B virus pre-S2 and S antigenicity, or a peptide having hepatitis B virus pre-S1, pre-S2 and S antigenicity.

12. A eukaryotic cell according to claim 8, wherein the peptide having hepatitis B virus surface antigenicity is a peptide having hepatitis B virus pre-S1, pre-S2 and S antigenicity.

13. A eukaryotic cell according to claim 8, which is Saccharomyces cerevisiae AH22R⁻/pGLD LP39-RcT.

14. A eukaryotic cell according to claim 8, which is Saccharomyces cerevisiae AH22R⁻/pGLD LIIP39-RcT.

15. A eukaryotic cell according to claim 8, which is Saccharomyces cerevisiae AH22R⁻/pGLD LP31-RcT.

16. A process for producing a peptide having hepatitis B virus surface antigenicity, which comprises (1) cultivating a eukaryotic cell which is transformed with a recombinant DNA in which a DNA coding for a signal peptide which functions in a eukaryotic cell is bound to the 5'-terminal of a DNA coding for a peptide having hepatitis B virus surface antigenicity, (2) accumulating the peptide having hepatitis B virus surface antigenicity in the culture medium and (3) recovering the peptide having hepatitis B virus surface antigenicity excreted in the culture medium.

17. A process according to claim 16, wherein the eukaryotic cell is a yeast cell.

18. A process according to claim 16, wherein the signal peptide is a signal peptide of egg white lysozyme.

19. A process according to claim 16, wherein the peptide having hepatitis B virus surface antigenicity is a peptide having hepatitis B virus S antigenicity, a peptide having hepatitis B virus pre-S2 and S antigenicity, or a peptide having hepatitis B virus pre-S1, pre-S2 and S antigenicity.

20. A process according to claim 16, wherein the peptide having hepatitis B virus surface antigenicity is a peptide having hepatitis B virus pre-S2 and S antigenicity.

21. A process for producing a peptide having hepatitis B virus pre-S1, pre-S2 and S antigenicity, which comprises (1) cultivating a eukaryotic cell which is transformed with a recombinant DNA in which a DNA coding for a signal peptide which functions in a eukaryotic cell is bound to the 5'-terminal of a DNA coding for a peptide having hepatitis B virus pre-S1, pre-S2 and S antigenicity, (2) accumulating the peptide having hepatitis B virus pre-S1, pre-S2 and S antigenicity in the culture and (3) recovering the peptide having hepatitis B virus pre-S1, pre-S2 and S antigenicity.

22. A process according to claim 21, wherein the eukaryotic cell is a yeast cell.

23. A process according to claim 21, wherein the signal peptide is a signal peptide of egg white lysozyme.

24. A process according to claim 21, wherein the transformed eukaryotic cell is Saccharomyces cerevisiae AH22R⁻/pGLD LP39-RcT.

25. A process according to claim 21, wherein the transformed eukaryotic cell is Saccharomyces cerevisiae AH22R⁻/pGLD LIIP39-RcT.

26. A process for producing a recombinant DNA in which a DNA coding for a signal peptide which functions in a eukaryotic cell is bound to the 5'-terminal of a DNA coding for a peptide having hepatitis B virus surface antigenicity, which comprises ligating the said DNA coding for the signal peptide to the 5'-terminal of the said DNA coding for the peptide having hepatitis B virus surface antigenicity.

27. A process according to claim 26, wherein the signal peptide is a signal peptide of egg white lysozyme.

28. A process according to claim 26, wherein the peptide having hepatitis B virus surface antigenicity is a peptide having hepatitis B virus S antigenicity, a peptide having hepatitis B virus pre-S2 and S antigenicity, or a peptide having hepatitis B virus pre-S1, pre-S2 and S antigenicity.

29. A process according to claim 26, wherein the peptide having hepatitis B virus surface antigenicity is a peptide having hepatitis B virus pre-S1, pre-S2 and S antigenicity.

30. A process according to claim 26, wherein the recombinant DNA is a plasmid replicable in a yeast cell.

31. A process for producing a eukaryotic cell which is transformed with a recombinant DNA in which a DNA coding for a signal peptide which functions in a eukaryotic cell is bound to the 5'-terminal of a DNA coding for a peptide having hepatitis B virus surface antigenicity, which comprises transforming a eukaryotic cell with the said recombinant DNA.

32. A process according to claim 31, wherein the eukaryotic cell is a yeast cell.

33. A process according to claim 31, wherein the signal peptide is a signal peptide of egg white lysozyme.

34. A process according to claim 31, wherein the peptide having hepatitis B virus surface antigenicity is a peptide having hepatitis B virus S antigenicity, a peptide having hepatitis B virus pre-S2 and S antigenicity, or a peptide having hepatitis B virus pre-S1, pre-S2 and S antigenicity.

35. A process according to claim 31, wherein the peptide having hepatitis B virus surface antigenicity is a peptide having hepatitis B virus pre-S1, pre-S2 and S antigenicity.

# Fig. 1

```
XhoI                MRSLLILVLCFLPLAALG                    EcoRI
|     #1     |           #3         |        #5        |       #17      |
TCGAGTATAAAAACAATGAGATCTTTGTTGATCTTGGTTTTGTGTTTCTTGCCATTGGCTGCTTTGGGTAAGGTTATGCAGTGG      (a)
   CATATTTTTGTTACTCTAGAAACAACTAGAACCAAAACACAAAGAACGGTAACCGACGAAACCCATTCCAATACGTCACCTTAA
 |     #2     |          #4          |        #6        |       #18      |


XhoI                M R S L L I L V L C F L P L A A L G        TaqI
|     #1    |           #3         |        #5       |    #7    |
TCGAGTATAAAAACAATGAGATCTTTGTTGATCTTGGTTTTGTGTTTCTTGCCATTGGCTGCTTTGGGTAAGGTTTT              (b)
   CATATTTTTGTTACTCTAGAAACAACTAGAACCAAAACACAAAGAACGGTAACCGACGAAACCCATTCCAAAAGC
 |     #2     |          #4          |        #6        |     #8    |
```

0 288 198

# Fig. 2

Fig. 3

# Fig. 4-(1)

# Fig. 4-(2)

# Fig. 5

```
1
ATG GGG ACG AAT CTT TCT GTT CCC AAT CCT CTG GGA TTC TTT CCC GAT CAC CAG TTG GAC
Met Gly Thr Asn Leu Ser Val Pro Asn Pro Leu Gly Phe Phe Pro Asp His Gln Leu Asp
21
CCT GCG TTC GGA GCC AAC TCA AAC AAT CCA GAT TGG GAC TTC AAC CCC AAC AAG GAT CAA
Pro Ala Phe Gly Ala Asn Ser Asn Asn Pro Asp Trp Asp Phe Asn Pro Asn Lys Asp Gln
41
TGG CCA GAG GCA AAT CAG GTA GGA GCG GGA GCA TTC GGG CCA GGG TTC ACC CCA CCA CAC
Trp Pro Glu Ala Asn Gln Val Gly Ala Gly Ala Phe Gly Pro Gly Phe Thr Pro Pro His
61
GGC GGT CTT TTG GGG TGG AGC CCT CAG GCT CAG GGC ATA TTG ACA ACA GTG CCA GCA GCA
Gly Gly Leu Leu Gly Trp Ser Pro Gln Ala Gln Gly Ile Leu Thr Thr Val Pro Ala Ala
81
CCT CCT CCT GCC TCC ACC AAT CGG CAG TCA GGA AGA CAG CCT ACT CCC ATC TCT CCA CCT
Pro Pro Pro Ala Ser Thr Asn Arg Gln Ser Gly Arg Gln Pro Thr Pro Ile Ser Pro Pro
101
CTA AGA GAC AGT CAT CCT CAG GCC ATG CAG TGG AAT TCC ACA ACA TTC CAC CAA GCT CTG
Leu Arg Asp Ser His Pro Gln Ala Met Gln Trp Asn Ser Thr Thr Phe His Gln Ala Leu
121
CTA GAT CCC AGA GTG AGG GGC CTA TAT TTT CCT GCT GGT GGC TCC AGT TCC GGA ACA GTA
Leu Asp Pro Arg Val Arg Gly Leu Tyr Phe Pro Ala Gly Gly Ser Ser Ser Gly Thr Val
141
AAC CCT GTT CCG ACT ACT GCC TCA CCC ATA TCT GGG GAC CCT GCA CCG AAC ATG GAG AAC
Asn Pro Val Pro Thr Thr Ala Ser Pro Ile Ser Gly Asp Pro Ala Pro Asn Met Glu Asn
161
ACA ACA TCA GGA TTC CTA GGA CCC CTG CTC GTG TTA CAG GCG GGG TTT TTC TTG TTG ACA
Thr Thr Ser Gly Phe Leu Gly Pro Leu Leu Val Leu Gln Ala Gly Phe Phe Leu Leu Thr
181
AGA ATC CTC ACA ATA CCA CAG AGT CTA GAC TCG TGG TGG ACT TCT CTC AAT TTT CTA GGG
Arg Ile Leu Thr Ile Pro Gln Ser Leu Asp Ser Trp Trp Thr Ser Leu Asn Phe Leu Gly
201
GGA GCA CCC ACG TGT CCT GGC CAA AAT TCG CAG TCC CCA ACC TCC AAT CAC TCA CCA ACC
Gly Ala Pro Thr Cys Pro Gly Gln Asn Ser Gln Ser Pro Thr Ser Asn His Ser Pro Thr
221
TCT TGT CCT CCA ATT TGT CCT GGC TAT CGC TGG ATG TGT CTG CGG CGT TTT ATC ATA TTC
Ser Cys Pro Pro Ile Cys Pro Gly Tyr Arg Trp Met Cys Leu Arg Arg Phe Ile Ile Phe
241
CTC TTC ATC CTG CTG CTA TGC CTC ATC TTC TTG TTG GTT CTT CTG GAC TAC CAA GGT ATG
Leu Phe Ile Leu Leu Leu Cys Leu Ile Phe Leu Leu Val Leu Leu Asp Tyr Gln Gly Met
261
TTG CCC GTT TGT CCT CTA CTT CCA GGA ACA TCA ACC ACC AGC ACG GGG CCA TGC AAG ACC
Leu Pro Val Cys Pro Leu Leu Pro Gly Thr Ser Thr Thr Ser Thr Gly Pro Cys Lys Thr
281
TGC ACG ATT CCT GCT CAA GGA ACC TCT ATG TTT CCC TCT TGT TGC TGT ACA AAA CCT TCG
Cys Thr Ile Pro Ala Gln Gly Thr Ser Met Phe Pro Ser Cys Cys Cys Thr Lys Pro Ser
301
GAC GGA AAC TGC ACT TGT ATT CCC ATC CCA TCA TCC TGG GCT TTC GCA AGA TTC CTA TGG
Asp Gly Asn Cys Thr Cys Ile Pro Ile Pro Ser Ser Trp Ala Phe Ala Arg Phe Leu Trp
321
GAG TGG GCC TCA GTC CGT TTC TCC TGG CTC AGT TTA CTA GTG CCA TTT GTT CAG TGG TTC
Glu Trp Ala Ser Val Arg Phe Ser Trp Leu Ser Leu Leu Val Pro Phe Val Gln Trp Phe
341
GTA GGG CTT TCC CCC ACT GTT TGG CTT TCA GTT ATA TGG ATG ATG TGG TAT TGG GGG CCA
Val Gly Leu Ser Pro Thr Val Trp Leu Ser Val Ile Trp Met Met Trp Tyr Trp Gly Pro
361
AGT CTG TAC AAC ATC TTG AGT CCC TTT TTA CCT CTA TTA CCA ATT TTC TTT TGT CTT TGG
Ser Leu Tyr Asn Ile Leu Ser Pro Phe Leu Pro Leu Leu Pro Ile Phe Phe Cys Leu Trp
381
GTA TAC ATT TAA
Val Tyr Ile ***
```

# Fig. 6

```
                        Met Glu Asn Ile Thr Ser Gly Phe Leu
                        ATG GAG AAC ATC ACA TCA GGA TTC CTA

Gly Pro Leu Leu Val Leu Gln Ala Gly Phe Phe Leu Leu Thr Arg Ile Leu Thr
GGA CCC CTG CTC GTG TTA CAG GCG GGG TTT TTC TTG TTG ACA AGA ATC CTC ACA

Ile Pro Gln Ser Leu Asp Ser Trp Trp Thr Ser Leu Asn Phe Leu Gly Gly Ser
ATA CCG CAG AGT CTA GAC TCG TGG TGG ACT TCT CTC AAT TTT CTA GGG GGA TCA

Pro Val Cys Leu Gly Gln Asn Ser Gln Ser Pro Thr Ser Asn His Ser Pro Thr
CCC GTG TGT CTT GGC CAA AAT TCG CAG TCC CCA ACC TCC AAT CAC TCA CCA ACC

Ser Cys Pro Pro Ile Cys Pro Gly Tyr Arg Trp Met Cys Leu Arg Arg Phe Ile
TCC TGT CCT CCA ATT TGT CCT GGT TAT CGC TGG ATG TGT CTG CGG CGT TTT ATC

Ile Phe Leu Phe Ile Leu Leu Leu Cys Leu Ile Phe Leu Leu Val Leu Leu Asp
ATA TTC CTC TTC ATC CTG CTG CTA TGC CTC ATC TTC TTA TTG GTT CTT CTG GAT

Tyr Gln Gly Met Leu Pro Val Cys Pro Leu Ile Pro Gly Ser Thr Thr Thr Ser
TAT CAA GGT ATG TTG CCC GTT TGT CCT CTA ATT CCA GGA TCA ACA ACA ACC AGT

Thr Gly Pro Cys Lys Thr Cys Thr Thr Pro Ala Gln Gly Asn Ser Lys Phe Pro
ACG GGA CCA TGC AAA ACC TGC ACG ACT CCT GCT CAA GGC AAC TCT AAG TTT CCC

Ser Cys Cys Cys Thr Lys Pro Thr Asp Gly Asn Cys Thr Cys Ile Pro Ile Pro
TCA TGT TGC TGT ACA AAA CCT ACG GAT GGA AAT TGC ACC TGT ATT CCC ATC CCA

Ser Ser Trp Ala Phe Ala Lys Tyr Leu Trp Glu Trp Ala Ser Val Arg Phe Ser
TCG TCC TGG GCT TTC GCA AAA TAC CTA TGG GAG TGG GCC TCA GTC CGT TTC TCT

Trp Leu Ser Leu Leu Val Pro Phe Val Gln Trp Phe Val Gly Leu Ser Pro Thr
TGG CTC AGT TTA CTA GTG CCA TTT GTT CAG TGG TTC GTA GGG CTT TCC CCC ACT

Val Trp Leu Ser Ala Ile Trp Met Met Trp Tyr Trp Gly Pro Ser Leu Tyr Ser
GTT TGG CTT TCA GCT ATA TGG ATG ATG TGG TAT TGG GGG CCA AGT CTG TAC AGC

Ile Val Ser Pro Phe Ile Pro Leu Leu Pro Ile Phe Phe Cys Leu Trp Val Tyr
ATC GTG AGT CCC TTT ATA CCG CTG TTA CCA ATT TTC TTT TGT CTC TGG GTA TAC

Ile ***
ATT TAA
```

# Fig. 7

```
XhoI                    M   R   S   L   L   I   L   V   L   C   F   L   P   L   A   A   L   G           TaqI
 |        #1        |                       #3              |                    #5           |            #27      |
    TCGAGTATAAAAACAATGAGATCTTTGTTGATCTTGGTTTTGTGTTTCTTGCCATTGGCTGCTTTGGGTAAGGTT
       CATATTTTTGTTACTCTAGAAACAACTAGAACCAAAACACAAAGAACGGTAACCGACGAAACCCATTCCAAGC
    |        #2        |                 #4                |                 #6              |        #28      |
```

# Fig. 8 -(1)

# Fig. 8 -(2)